(19) Europäisches Patentamt
European Patent Office
Office européen des brevets

(11) Numéro de publication : 0 387 157 B1

(12) # FASCICULE DE BREVET EUROPEEN

(45) Date de publication du fascicule du brevet : **27.07.94 Bulletin 94/30**

(51) Int. Cl.$^5$ : **C08J 3/03,** C08L 83/04, C08K 13/02

(21) Numéro de dépôt : **90420054.0**

(22) Date de dépôt : **01.02.90**

(54) **Dispersion aqueuse de silicone à base d'aminosilane et/ou d'amidosilane réticulant et un élastomère par élimination de l'eau.**

(30) Priorité : **03.02.89 FR 8901654**

(43) Date de publication de la demande : **12.09.90 Bulletin 90/37**

(45) Mention de la délivrance du brevet : **27.07.94 Bulletin 94/30**

(84) Etats contractants désignés : **AT BE CH DE DK ES FR GB GR IT LI LU NL SE**

(56) Documents cités :
**EP-A- 0 143 541**
**EP-A- 0 201 284**
**FR-A- 2 195 655**

(73) Titulaire : **RHONE-POULENC CHIMIE**
**25, quai Paul Doumer**
**F-92408 Courbevoie Cédex (FR)**

(72) Inventeur : **Feder, Michel**
**35, rue du Séminaire,**
**Zillisheim**
**F-68720 Illfurth (FR)**

(74) Mandataire : **Trolliet, Maurice et al**
**RHONE-POULENC CHIMIE**
**Direction de la Propriété Industrielle**
**Centre de Recherches des Carrières**
**B.P. 62**
**F-69192 Saint-Fons Cédex (FR)**



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne une dispersion aqueuse de silicone à base d'aminosilane et/ou d'amidosilane pouvant réticuler en un élastomère par élimination de l'eau.

Le brevet US-A-2 891 920 décrit un procédé de polymérisation en émulsion de polydiorganosiloxane en utilisant un catalyseur acide ou basique en présence d'agents tensio-actifs anioniques, cationiques ou non-ioniques. Ce brevet enseigne que les émulsions obtenues sont stables au stockage et sont utiles, après addition de charges, pour faire des peintures donnant un revêtement continu par élimination de l'eau.

Le brevet US-A-3 294 725 décrit en particulier l'utilisation d'acide dodécylbenzène-sulfonique pour polymériser en émulsion des polydiorganosiloxanes. Ce brevet enseigne que pour obtenir des émulsions stables, il est souhaitable de règler le pH de ces émulsions à une valeur de 7 environ. Ce brevet enseigne que l'on peut obtenir à partir de ces émulsions neutralisées, auxquelles on a ajouté de la silice colloïdale et un polyalcoxysilane, un revêtement élastomère.

L'enseignement du brevet US-A-3 360 491 est similaire à celui de US-A-3 294 725, sauf que l'acide dodécylbenzène sulfonique est remplacé par l'hydrogénolaurylsulfate.

Le brevet US-A-3 697 496 décrit un procédé particulier de polymérisation en émulsion de polydiorganosiloxanes et indique la possibilité d'ajouter à cette émulsion de la silice colloïdale et un sel d'étain en vue d'obtenir un revêtement élastomère par évaporation d'eau.

Le brevet français FR-A-2 110 358 décrit une émulsion silicone à pH compris entre 6,5 et 9, réticulant en un élastomère conducteur de l'électricité après évaporation de l'eau par incorporation de noir de carbone. L'émulsion, comportant en outre un sel d'étain et un polyalcoxysilane, n'est pas stable au stockage et doit être conservée en deux emballages distincts (émulsion bicomposante).

Les brevets américains US-A-4 221 688, US-A-4 244 849 et français FR-A-2 463 163 décrivent des émulsions silicone stables au stockage et comportant :

- une émulsion d'un polymère $\alpha,\omega$-(dihydroxy)polydiorganosiloxane stabilisée anioniquement,
- une charge siliceuse,
- un sel d'étain,
- éventuellement une charge non renforçante.

La charge siliceuse peut être une silice colloïdale (US-A-4 221 688), du silicate de soude (US-A-4 244 849), ou une silice amorphe en poudre (FR-A-2 463 163).

Par rapport aux émulsions (dispersions) aqueuses connues de l'art antérieur, ces trois brevets enseignent d'une part que pour obtenir une émulsion monocomposante stable au stockage il faut conserver l'émulsion à un pH alcalin supérieur à 8,5 ou 9, de préférence supérieur à 10, et, d'autre part, incorporer à l'émulsion un sel d'étain pour raccourcir à quelques jours l'étape de maturation de l'émulsion nécessaire à l'obtention d'une dispersion capable de réticuler.

Le brevet US-A-3 355 406 enseigne un latex de silicone constitué d'un $\alpha,\omega$-(dihydroxy)polydiorganosiloxane, de préférence préparé par polymérisation en émulsion et d'une résine silsesquioxane constituée de motifs $RSiO_{1,5}$ (R reste hydrocarboné). Le latex peut comporter en outre un catalyseur métallique de durcissement et un alkyltrialcoxysilane.

Dans le brevet US-A-4 554 187, la résine silicone associée à l'$\alpha,\omega$-(dihydroxy)polydiorganosiloxane est une résine réactive de faible poids moléculaire présentant des groupes alcoxy ou acyloxy.

Dans la demande de brevet EP-A-266 729 la résine silicone, associée à l'$\alpha,\omega$-(dihydroxy)polydiorganosiloxane et au catalyseur de durcissement, est un siliconate.

A ce siliconate peut être associé une résine silicone comportant jusqu'à 10 % en poids de groupe hydroxyle.

Le brevet US-A-4,618,642 (EP-A-201 284) enseigne une dispersion aqueuse de silicone comportant une charge non siliceuse, un alcoxysilane ou un cétiminoxysilane comme agent réticulant et un agent d'adhérence du type gamma-aminopropyltriéthoxysilane. D'après US-A-4,608,412, un orthosilicate d'alkyleest également utilisable. Les dispersion aqueuses obtenues présentent toutefois une stabilité au stockage insuffisante.

L'utilisation d'alcoxysilane associé à une charge siliceuse est par ailleurs décrite par US-A-4,618,645.

Le brevet EP-A-143 541 vise une dispersion aqueuse de silicone comportant de la silice colloïdale ou un silicate de métal alcalin, dispersion à laquelle est ajouté après mâturation un agent d'adherence améliorant la brillance , du type gamma-aminopropyltriméthoxysilane.

Les aminosilanes et les amidosilanes sont des produits connus dont l'association, à l'abri de l'humidité de l'air avec un $\alpha,\omega$-(dihydroxy)diorganopolysiloxane et un catalyseur de durcissement, conduit à une composition monocomposante stable au stockage en absence d'eau et réticulant en un élastomère en présence de l'humidité de l'air comme l'enseignent par exemple FR-A-1 248 826 et FR-A-1 423 477.

Toutefois ces deux brevents ne décrivent, ni ne suggèrent, que la composition silicone puisse être sous

la forme d'une dispersion aqueuse.

En outre les documents visant des dispersions aqueuses de silicone ne décrivent pas l'utilisation d'aminosilane et/ou d'amidosilanes en association avec une émulsion aqueuse d'un $\alpha,\omega$-(dihydroxy)diorganopolysiloxane et, éventuellement un catalyseur métallique de durcissement.

Un but de la présente invention est de proposer une dispersion aqueuse de silicone qui soit stable au stockage pendant plusieurs mois, qui réticule correctement et suffisamment rapidement en un élastomère par élimination de l'eau à température ambiante, l'élastomère formé conservant ses propriétés mécaniques lors de son vieillissement.

Un autre but est de proposer une dispersion aqueuse de silicone du type ci-dessus dont l'étape de maturation puisse être effectuée à une température peu élevée (20-60 °C) et pendant une durée inférieure à 48 heures.

Un autre but est de proposer une dispersion aqueuse de silicone du type ci-dessus qui puisse comporter, en plus de charges inertes, des charges basiques et des charges acides.

Un autre but est de proposer une dispersion aqueuse de silicone du type ci-dessus conduisant à un élastomère présentant en outre une résistance à la flamme améliorée.

Un autre but est de proposer une dispersion aqueuse de silicone du type ci-dessus conduisant à un élastomère qui puisse présenter une adhérence satisfaisante sur divers supports en particulier sur verre, béton et métaux (acier, aluminium).

Ces buts et d'autres sont atteints par la présente invention qui concerne en effet une dispersion aqueuse de silicone, réticulant en un élastomère par élimination de l'eau dans les conditions ambiantes, caractérisée en ce qu'elle comporte :

(A) - 100 parties en poids d'une émulsion du type huile dans eau d'un $\alpha,\omega$-(dihydroxy)polydiorganosiloxane stabilisée par au moins un agent tensio-actif choisi parmi les agents tensio-actifs anioniques et non-ioniques et leurs mélanges,

(B) - 0,1 à 20 parties en poids d'un silane de formule :

$$(R^2O)_{4-(a+b)}\overset{\displaystyle (R^1)_b \atop |}{Si}-(X)_a \qquad (1)$$

dans laquelle

- X est un groupe hydrolysable choisi parmi un radical amino de formule $R^3NH$- et amido de formule $R^4$-CO-N($CH_3$)-
  - $R^1$ est un radical hydrocarboné monovalent en $C_1$-$C_{13}$
  - $R^2$ est un radical organique aliphatique en $C_1$-$C_8$ choisi parmi les radicaux alkyle, les radicaux alkyléther, les radicaux alkylester, les radicaux cyanoalkyle ou un radical aralkyle en $C_7$-$C_{13}$,
- $R^3$ est un radical alkyle en $C_1$-$C_6$, phényle ou cyclohexyle
- $R^4$ est un radical alkyle en $C_1$-$C_6$ ou phényle
  - a est un nombre entier égal à 2, 3 ou 4,
  - b est un nombre entier égal à 0 ou 1,
  - a + b est égal à 2, 3 ou 4,

(C) - 0 à 3 parties en poids d'un composé catalytique métallique de durcissement,

(D) - 0 à 250 parties en poids d'une charge minérale non siliceuse,

ladite dispersion ayant un pH supérieur à 7, de préférence compris entre 8 et 13, et une teneur en extraits secs d'au moins 40 %.

Les $\alpha,\omega$-(dihydroxy)polydiorganosiloxane doivent avoir une viscosité d'au moins 100 mPa.s à 25 °C, de préférence d'au moins 50 000 mPa.s.

C'est en effet pour des viscosités supérieures à 50 000 mPa.s que l'on obtient un élastomère présentant un ensemble de propriétés mécaniques convenables, en particulier au niveau de la dureté Shore A et de l'allongement.

En outre plus la viscosité est élevée et plus les propriétés mécaniques se conservent lors du vieillissement de l'élastomère.

Les viscosités préférées pour la présente invention sont comprises entre 50 000 et 1 500 000 mPa.s à 25 °C.

Les radicaux organiques des $\alpha,\omega$-(dihydroxy)polydiorganopolysiloxanes sont des radicaux hydrocarbonés

monovalents contenant jusqu'à 6 atomes de carbone, éventuellement substitué par des groupes cyano ou fluoro. Les substituants généralement utilisés du fait de leur disponibilité dans les produits industriels sont les radicaux méthyle, éthyle, propyle, phényle, vinyle et 3,3,3-trifluoropropyle. En général au moins 80 % en nombre de ces radicaux sont des radicaux méthyle.

Dans le cadre de la présente invention on préfère plus spécialement utiliser les $\alpha,\omega$-(dihydroxy)polydiorganosiloxanes préparés par le procédé de polymérisation anionique décrit dans les brevets américains précités : US-A-2891 920 et surtout US-A-3 294 725 (cités comme référence). Le polymère obtenu est stabilisé anioniquement par un agent tensio-actif qui, conformément à l'enseignement de US-A-3 294 725 est de préférence le sel d'un métal alcalin d'un acide hydrocarboné aromatique sulfonique, l'acide libre jouant également le rôle de catalyseur de polymérisation en emulsion.

Le catalyseur de polymerisation en émulsion et l'agent tensio-actif préférés sont l'acide dodécylbenzène-sulfonique et ses sels de métaux alcalins, en particulier son sel de sodium. On peut ajouter éventuellement d'autres agents tensio-actifs anioniques ou non-ioniques. Toutefois cet ajout n'est pas nécessaire car, conformément à l'enseignement de US-A-3 294 725, la quantité d'agent tensio-actif anionique résultant de la neutralisation de l'acide sulfonique est suffisante pour stabiliser l'émulsion de polymère. Cette quantité est généralement inférieure à 3 %, de préférence 1,5 % du poids de l'émulsion.

Ce procédé de polymérisation en émulsion est particulièrement intéressant car il permet d'obtenir directement l'émulsion (A). Par ailleurs ce procédé permet de pouvoir obtenir sans difficulté des émulsions (A) de $\alpha,\omega$-(dihydroxy)polydiorganosiloxane de très haute viscosité.

Pour préparer l'émulsion (A) on peut également partir d'$\alpha,\omega$-(dihydroxy)polydiorganosiloxane déjà polymérisé, puis le mettre en émulsion aqueuse en stabilisant les émulsions par un agent tensio-actif anionique et/ou non-ionique suivant un procédé bien connu de l'homme de métier et décrit en détail dans la littérature (voir par exemple les brevets FR-A-2 064 563, FR-A-2 094 322, FR-A-2 114 230 et EP-A-169 098).

Selon ce procédé, on mélange par simple agitation les polymères $\alpha,\omega$-(dihydroxy)polydiorganosiloxane avec l'agent tensio-actif anionique ou non-ionique, ce dernier pouvant être en solution aqueuse, à ajouter ensuite si nécessaire de l'eau et à transformer l'ensemble en une émulsion fine et homogène par passage dans un broyeur classique à colloïdes.

Par la suite le broyat obtenu est dilué par une quantité d'eau appropriée et on obtient ainsi une émulsion (A) stabilisée par un agent tensio-actif anionique on non-ionique stable au stockage.

La quantité d'agent tensio-actif anionique et non-ionique utilisable est celle utilisée couramment pour la mise en oeuvre du procédé de mise en émulsion, en particulier ceux décrits dans les brevets précités et dans le brevet US-A-2 891 920.

Dans le cadre de la présente invention les agents tensio-actifs anioniques préférés sont le sel d'un métal alcalin d'un acide hydrocarboné aromatique sulfonique et les agents tensio-actifs non-ioniques préférés sont les alkylphénols polyoxyéthylénés. Ces agents tensio-actifs non-ioniques sont bien entendu les mêmes que ceux que l'on peut ajouter éventuellement aux émulsions (A) obtenues par polymérisation en émulsion comme indiqué plus haut.

L'émulsion (A) préparée par polymérisation en émulsion ou par mise en émulsion du polymère silicone se présente sous la forme d'une émulsion huile dans l'eau et a, de préférence, une teneur en extrait sec supérieure à 45 % en poids.

Pour 100 parties d'émulsion (A), on incorpore de 0,1 à 20, de préférence de 0,5 à 10, parties, de silane (B) de formule (1).

Les aminosilanes de formule (1) sont en particulier décrits dans FR-A-1 248 826 précité.

Les amidosilanes de formule (1) sont en particulier décrits dans FR-A-1 423 477 précité.

Les radicaux $R^1$ de la formule (1) incluent les radicaux alkyle et alcényle tels que méthyle, éthyle, propyle, vinyle, allyle, trifluoro-3,3,3-propyle et cyano-éthyle, des radicaux aryle tel que phényle, et cycloalkyle tel que cyclohexyle.

Les radicaux $R^2$ de la formule (1) incluent les radicaux alkyle en $C_1$-$C_8$ tels que méthyle, éthyle, propyle, éthyl-2 hexyle, les radicaux aralkyle tel que benzyle, les radicaux alkyléther tel que méthoxy-2 éthyle, les radicaux cyanoalkyl tel que cyano-2 éthyle et les radicaux alkylester tel que le radical acétoxy-2 éthyl.

Dans la formule (1), les radicaux $R^1$, $R^2$, $R^3$, $R^4$ et $R^5$ peuvent être identiques ou différents.

Comme aminosilanes utilisables on peut citer :

- méthyl tris(n-butylamino)silane,
- méthyl tris {(éthyl-2) hexylamino}}silane,
- phényl tris(iso-butylamino)silane,
- méthyl tris(cyclohexylamino)silane,
- phényl tris(éthylamino)silane,
- méthylméthoxy di(-N-méthylamino)silane,

- tétra (N-N-diéthylamino)silane,
- méthyl tris (phénylamino)silane.

Comme amidosilanes utilisables, on peut citer :
- méthyl tris(N-méthylacétamido)silane,
- méthyl tris (N-méthylbenzamido)silane,
- méthyl méthoxy bis(N-méthylacétamido)silane,
- méthyléthoxy bis(N-méthylbenzylamino)silane.

Les silanes (B) et leurs produits d'hydrolyse partielle agissent comme agents de réticulation dans les dispersions aqueuses de silicone selon l'invention.

Les composés catalytiques métalliques de durcissement (C) ne sont pas nécessaires surtout si on utilise certaines charges basiques (D) mentionnées ci-dessous. Cependant ces catalyseurs permettent d'accélérer considérablement la maturation du système. Les catalyseurs utilisables de préférence sont essentiellement les sels d'acides carboxyliques et les halogénures de métaux choisis parmi le plomb, le zinc, le zirconium, le titane, le fer, l'étain, le baryum, le calcium et le manganèse.

Le constituant (C) est de préférence un composé catalytique à l'étain, généralement un sel d'organoétain, introduit de préférence sous forme d'une émulsion aqueuse. Les sels d'organoétain utilisables sont décrits, en particulier dans l'ouvrage de NOLL, Chemistry and Technology of Silicones Academic Press (1968), page 337.

On peut également utiliser comme composé catalytique à l'étain le produit de réaction d'une sel d'étain, en particulier d'un di-carboxylate d'étain sur du polysilicate d'éthyle, comme décrit dans le brevet US-A-3 862 919.

On peut également utiliser le produit de réaction d'un silicate d'alkyle ou d'un alkyltrialcoxysilane sur le diacétate de dibutylétain comme décrit dans le brevet belge BE-A-842 305.

Les sels d'étain préférés sont les bischélates d'étain (EP-A-147 323 et EP-A-235 049), les dicarboxylates de diorganoétain et en particulier les diversatates de dibutyl- ou de dioctylétain (brevet britannique GB-A-1 289 900), le diacétate de dibutyl- ou de dioctylétain, le dilaurate de dibutyl- ou de dioctylétain. On utilise de 0,01 à 3, de préférence, de 0,05 à 2 parties de sel d'organoétain pour 100 parties de (A).

Un autre constituant de la dispersion selon l'invention est l'addition de 0 à 250, de préférence, de 5 à 200 parties d'une charge minérale non siliceuse semi-renforçante ou de bourrage (D).

Les charges (D) ont une granulométrie généralement comprise entre 0,001 et 300 $\mu$m et une surface BET inférieure à 100 $m^2$/g.

Des exemples de charges (D) utilisables seules ou en mélange sont le noir de carbone, le dioxyde de titane, l'oxyde d'aluminium, l'alumine hydratée, la vermiculite expansée, la vermiculite non expansée, le carbonate de calcium, le carbonate de zinc, le carbonate de magnésium, la magnésie, l'oxyde de zinc, le mica, le talc, l'oxyde de fer, le sulfate de baryum et la chaux éteinte.

Certaines de ces charges peuvent accélérer de façon sensible le durcissement de la dispersion et jouer, en totalité ou en partie, le rôle du catalyseur (C).

Ces charges (D) sont introduites dans l'émulsion (A) sous forme de poudre sèche par exemple par simple mélange.

L'invention vise tout particulièrement un dispersion aqueuse de silicone comportant:

(A) - 100 parties de l'émulsion du type huile dans eau d'un $\alpha,\omega$-(dihydroxy)polydiorganosiloxane de viscosité à 25 °C comprise entre 50 000 et 1 500 000 mPa.s et est stabilisée par un agent tensio-actif choisi parmi un sel de métal alcalin d'un acide hydrocarboné aromatique sulfonique et les alkylphénols polyoxyéthylénés,

(B) - 0,5 à 10 parties de silane,

(C) - 0,05 à 2 parties d'un dicarboxylate de diorganoétain,

(D) - 10 à 200 parties d'une charge minérale non siliceuse ladite émulsion ayant un pH compris entre 8 et 13 et une teneur en extraits secs d'au moins 60 %.

Selon une variante de l'invention, on a découvert que si la charge (D) n'est sensiblement constituée que d'une charge choisie parmi l'alumine hydratée, la vermiculite expansée, la vermiculite non expansée, selon une teneur de 5 à 250, de préférence de 10 à 200 parties pour 100 parties d'émulsion (A) on obtient un élastomère ayant une résistance à la flamme particulièrement élevée qui ne peut pas être obtenue avec les autres catégories de charge (D) précitées, en particulier avec l'oxyde d'aluminium ou alumine non hydratée. On peut également incorporer des fibres céramiques ou d'aramide selon l'enseignement de EP-A-212 827.

Selon une variante, on peut incorporer en outre, pour 100 parties d'émulsion (A) un additif silicié (E) choisi parmi du silicate de soude (0,3 à 30 parties), et une charge siliceuse renforçante ou semi-renforçante (1 à 150 parties).

Ces charges siliceuses sont choisies parmi la silice colloïdale, les poudres de silice de combustion et de

précipitation ou leur mélange. La silice de combustion est préférée. On peut toutefois utiliser également des charges siliceuses semi-renforçantes telles que des terres de diatomées, du quartz broyé.

Pour 100 parties d'émulsion (A), la somme des parties de (D) + (E) doit être inférieure à 300 parties.

Les poudres de silice de combustion et de précipitation sont bien connues, elles sont utilisées en particulier comme charges dans les compositions élastomère de silicone, vulcanisables à chaud et un caoutchouc de silicone. Ces poudres présentent une taille moyenne de particules généralement inférieures à 0,1 μm et une surface spécifique BET supérieure à 50 $m^2/g$, de préférence comprise entre 150 et 350 $m^2/g$.

L'incorporation dans l'émulsion (A) de cet additif silicié (E), par tout moyen convenable, en particulier par agitation, augmente considérablement la viscosité de l'émulsion (A) qui présente alors un caractère pâteux.

On a en effet trouvé, conformément à la présente invention que l'addition de cet additif silicié (E), est suffisante pour conférer à la dispersion un caractère "thixotrope" plus ou moins marqué. L'émulsion, extraite par exemple d'une cartouche de stockage, adhère sans s'écouler sur un substrat même vertical et durcit en élastomère par évaporation de l'eau à température ambiante. On peut également obtenir une émulsion non coulante en utilisant comme charge (D) du carbonate de calcium dont le diamètre particulaire moyen est inférieur à 0,1 μm. Bien entendu un léger chauffage (à 40-80 °C environ) de la composition pour accélérer l'évaporation de l'eau n'est pas exclu du cadre de l'invention.

Pour 100 parties d'émulsion (A), on peut incorporer en outre de 1 à 40, de préférence de 2 à 20 parties, calculées en extraits secs, d'une résine silicone hydroxylée (F).

La résine silicone hydroxylée (F) possède une teneur pondérale en groupe hydroxyle comprise entre 0,1 et 10 %, de préférence entre 1 et 6 %.

Cette résine (F) présente, par molécule, au moins deux motifs différents choisis parmi ceux de formules : $R_3SiO_{0,5}$ (motif M), $R_2SiO$ (motif D), $RSiO_{1,5}$ (motif T) et $SiO_2$ (motif Q).

Les motifs M, D, T et Q sont distribués de telle façon que le rapport molaire R/Si soit inférieur à 2, de préférence inférieur à 1,8, pour exclure les polydiorganosiloxanes linéaires.

Les radicaux R, identiques ou différents, sont choisis parmi les radicaux vinyle, phényle, trifluoro-3,3,3 propyle et les radicaux alkyle linéaires ou ramifiés ayant de 1 à 6 atomes de carbone inclus.

Comme exemples de radicaux R alkyle on peut citer les radicaux méthyle, éthyle, isopropyle, tertiobutyle et n-hexyle.

Ces résines silicones sont des polymères organopolysiloxanes ramifiés bien connus dont les procédés de préparation sont décrits dans de très nombreux brevets.

Comme exemples de résines utilisables on peut citer les résines MQ, les résines MQD, les résines TD et les résines MDT.

On peut utiliser les résines qui sont solides ou liquides à température ambiante. Ces résines peuvent être incorporées dans les émulsions aqueuses telles quelles, en solution dans un solvant organique ou une huile silicone, on bien sous forme d'émulsion aqueuses.

Des émulsions aqueuses de résines silicones utilisables sont par exemple décrites dans les brevets US-A-4 028 339, US-A-4 052 331, US-A-4 056 492, US-A-4 525 502 et US-A-4 717 599, cités comme référence.

Selon un mode de réalisation préféré de l'invention, on peut améliorer grandement l'adhérence sur divers supports des élastomères issus des dispersions selon l'invention en réglant le pH de la dispersion a une valeur comprise entre 8 et 13 en ajoutant une quantité adaptée d'une solution aqueuse d'une base minérale forte (G) choisie parmi les hydroxydes de métaux alcalins ou alcalino-terreux tels que la soude, la potasse et l'hydroxyde de calcium, l'hydroxyde de magnésium et l'hydroxyde de baryum.

Comme autres exemples d'additifs à incorporer éventuellement aux dispersions selon l'invention, on peut citer les antigels, les antifongiques, les antimousses ainsi que les agents thixotropants comme la carboxyméthylcellulose, la gomme xanthane et l'alcool polyvinylique.

Les dispersions selon l'invention peuvent être préparées de préférence de la façon suivante :

On part d'une émulsion (A) préparée soit par le procédé de polymérisation en émulsion et l'on dispose d'une émulsion stabilisée par un agent tensio-actif anionique et éventuellement non-ionique, soit par le procédé de mise en émulsion de l'$\alpha,\omega$-(dihydroxy)polydiorganosiloxane et l'on dispose d'une émulsion stabilisée par un agent tensio-actif anionique et/ou non-ionique.

Pour préparer les dispersions selon l'invention, il est recommandé d'ajuste tout d'abord, à température ambiante, le pH de l'émulsion (A), à une valeur comprise entre 7 et 13, de préférence entre 8 et 13, au moyen d'une base organique (par exemple la diéthylamine) ou minérale {additif (G)}. L'additif (G) est préféré car il permet d'obtenir un élastomère adhérant mieux sur les supports.

On ajoute ensuite éventuellement le catalyseur (C), puis le silane (B) et éventuellement la charge (D) et (E) ainsi que la résine (F).

La résine (F) est ajoutée telle quelle, ou en solution dans un solvant organique ou dans une huile silicone, ou bien sous forme d'une émulsion aqueuse.

Comme huile silicone on peut utiliser un polydiméthylsiloxane bloqué triméthyle silyle, de viscosité à 25 °C comprise entre 100 et 5 000 mPa.s.

L'émulsion finale obtenue est homogénéisée puis dégazée et est ensuite conditionnée en emballage étanche à l'oxygène de l'air et à la vapeur d'eau.

Les constituants (A), (B), et éventuellement (C), (D), (E), (F) et (G) sont mélangés en des quantités telles que l'émulsion finale présente une teneur en extrait sec supérieure à 40 %, de préférence supérieure à 60 %, mais généralement inférieure à 90 %. La zone de pH préférée est comprise entre 8 et 13.

Les dispersions selon l'invention peuvent être utilisées comme peinture réticulable en couche mince. Elles présentent alors, de préférence, un extrait sec compris entre 40 et 70 %.

Pour déterminer la teneur en extrait sec on place 2 g de dispersion dans une coupelle de pesage en aluminium et on la chauffe une heure à 150 °C dans un four à circulation d'air. Après refroidissement on pèse à nouveau la coupelle et on détermine le pourcentage de matière restante sur les 2 g initiaux qui représente la teneur en extrait sec.

Selon une variante préférée, la dispersion selon l'invention après sa préparation subit une étape de maturation, à température ambiante, de quelques heures à quelques jours.

Cette étape de maturation consiste simplement à laisser reposer la dispersion à l'abri de l'oxygène de l'air avant son utilisation.

Les dispersions selon l'invention peuvent être utilisées pour la réalisation de joints élastomères silicone, en particulier pour le bâtiment.

Ces dispersions sont également utilisables pour l'enrobage de divers principes actifs pharmaceutiques ou phytosanitaires formulés sous une forme solide (pastilles, tablettes, pillules, etc .....), pour l'enduction de bouchons de liège utilisés pour obturer les bouteilles de vins et de spiritueux, pour réaliser des revêtements d'objets culinaires et, de façon générale, d'objets en contact avec des aliments (par exemple des moules à pains).

Les techniques connues d'enrobage sont utilisables en particulier les techniques d'enduction au pinceau et au trempé (par immersion), les techniques de pulvérisation, les techniques d'enrobage en lit fluidisé et les techniques d'enduction par immersion.

Pour les revêtements de bouchons de liège, une technique recommandée est la technique au trempé qui consiste à immerger les bouchons dans la dispersion qui mouille la surface du bouchon, puis à évaporer l'eau.

Le revêtement obtenu représente 20 à 50 mg d'élastomère pour 100 $cm^2$ de surface de bouchon. Cette couche facilite le glissement du bouchon dans le goulot de la bouteille lors de l'embouteillage et évite le "coulage", c'est-à-dire des fuites de liquide entre le goulot et le bouchon.

Dans ce qui suit ou ce qui précède, sauf mentions contraires, les pourcentages et parties sont en poids.

- EXEMPLE 1 :

Préparation de l'émulsion (A) : elle est obtenue par polymérisation en émulsion d'huile $\alpha,\omega$-(dihydroxy)polydiméthylsiloxane de viscosité 100 mPa.s à 25 °C en présence d'acide dodécylbenzène sulfonique.

Quand la viscosité de l'huile atteint $10^6$ mPa.s à 25 °C, on arrête la polymérisation par neutralisation du catalyseur.

L'émulsion (A) obtenue a une teneur en extrait sec de 59 %.

A 100 parties d'émulsion (A) on ajoute sous agitation 2,2 parties d'une solution aqueuse de potasse (G) à 30 %, puis 1,5 partie d'émulsion aqueuse (C) à 37 % en poids de dilaurate de di-noctylétain, 4,1 parties de méthyléthoxy bis(N-méthylbenzamido)silane (B) et 58,5 parties de poudre de $CaCO_3$ (D) de précipitation , de granulométrie moyenne de 70 nanomètres.

Les constituants (A), (B), (C) (D) et (G) sont ajoutés dans l'ordre indiqué à température ambiante, et en respectant pour chaque réactif une durée d'incorporation de 15 minutes environ.

La dispersion finale a une teneur en extrait sec de 70 % et présente directement un pH de 9.

La dispersion finale obtenue est homogénéisée pendant 30 minutes sous vide puis conditionnée sous un emballage étanche à l'oxygène de l'air et à la vapeur d'eau.

Après 7 jours de stockage, on étend à la râcle la dispersion suivant une pellicule (film) de 2 mm d'épaisseur qu'on laisse sécher pendant 7 jours à température ambiante (20 °C) pour un premier lot et pendant 3 mois à température ambiante pour un deuxième lot.

L'aspect, l'extrusion et la réactivité des dispersions ne sont que très peu modifiées après 3 mois de stockage.

Sur les pellicules séchées on mesure les propriétés mécaniques moyennes suivantes :

- la dureté Shore A (DSA) selon la norme ASTM-D-2240,
- la résistance à la rupture (R/R) selon la norme AFNORT-T 46 002 correspondant à la norme ASTMD

412, en MPa.s,
- l'allongement à la rupture (A/R) en % selon la norme AFNOR-T 46 002,
- le module élastique (ME) à 100 % d'allongement selon la norme AFNOR-T 46 002, en MPa.

Les propriétés mécaniques obtenues sont rassemblées dans le tableau ci-après.

Pour apprécier l'adhérence, on dépose sur un support en verre ou en béton 1 cordon de dispersion aqueuse de 4 mm d'épaisseur. Après 12 jours on apprécie l'adhérence de l'élastomère formé en tirant manuellement sur le cordon.

Les adhésions ont été qualifiées de trois manières :
- bonne adhérence lorsque le cordon ne peut être décollé de son support (notée ++),
- adhérence moyenne lorsque le cordon est décollé difficilement et par petites surfaces (notée +),
- absence d'adhérence lorsque le cordon se décolle facilement (notée 0).

Les propriétés mécaniques et les appréciations de l'adhésion sont rassemblées dans le tableau ci-après.

- EXEMPLE 2 :

On répète exactement le mode opératoire de l'exemple 1 sauf qu'on ajoute 4,1 parties de méthyl tris(cyclohexylamino)silane à la place des 4,1 parties de benzamidosilane.

Le pH de la dispersion finale est de 9.

L'émulsion finale a une teneur en extraits secs de 71 %.

Les propriétés mécaniques et les appréciations de l'adhésion sont rassemblées dans le tableau ci-après.

TABLEAU

| EXEMPLES | | 1 | 2 |
|---|---|---|---|
| DSA | 7 jours à 20 °C | 30 | 20 |
| | 3 mois à 20 °C | 27 | 18 |
| R/R (MPa) | 7 jours à 20 °C | 1,05 | 0,9 |
| | 3 mois à 20 °C | 1,00 | 0,7 |
| A/R (%) | 7 jours à 20 °C | 468 | 713 |
| | 3 mois à 20 °C | 440 | 685 |
| MR (MPa) | 7 jours à 20 °C | 0,46 | 0,31 |
| | 3 mois à 20 °C | 0,44 | 0,30 |
| ADHERENCE VERRE | | xx | xx |
| ADHERENCE BETON | | + | + |

## Revendications

1. Dispersion aqueuse de silicone, réticulant en un élastomère par élimination de l'eau dans les conditions ambiantes, caractérisée en ce qu'elle comporte, en poids :
(A) - 100 parties en poids d'une émulsion du type huile dans eau d'un α,ω-(dihydroxy)polydiorganosiloxane stabilisée par au moins un agent tensio-actif choisi parmi les agents tensio-actifs anioniques et non-ioniques et leurs mélanges,
(B) - 0,1 à 20 parties en poids d'un silane de formule :

$$(R^2O)_{4-(a+b)}\overset{\displaystyle (R^1)_b \atop |}{Si}-(X)_a \qquad (1)$$

dans laquelle
- X est un groupe hydrolysable choisi parmi un radical amino de formule $R^3NH$- et amido de formule $R^4$-CO-N($CH_3$)-
  - $R^1$ est un radical hydrocarboné monovalent en $C_1$-$C_{13}$
  - $R^2$ est un radical organique aliphatique en $C_1$-$C_8$ choisi parmi les radicaux alkyle, les radicaux alkyléther, les radicaux alkylester, les radicaux cyanoalkyle ou un radical aralkyle en $C_7$-$C_{13}$,
- $R^3$ est un radical alkyle en $C_1$-$C_6$, phényle ou cyclohexyle
- $R^4$ est un radical alkyle en $C_1$-$C_6$ ou phényle
  - a est un nombre entier égal à 2, 3 ou 4,
  - b est un nombre entier égal à 0 ou 1,
  - a + b est égal à 2, 3 ou 4,

(C) - 0 à 3 parties en poids d'un composé catalytique métallique de durcissement,
(D) - 0 à 250 parties en poids d'une charge minérale non siliceuse,
ladite dispersion ayant un pH supérieur à 7 et une teneur en extrait sec d'au moins 40 %.

2. Dispersion aqueuse selon la revendication 1, caractérisée en ce qu'elle comporte 0,01 à 3 parties de composé métallique (C) qui est un sel d'organoétain (D) se trouvant sous forme d'une émulsion aqueuse.

3. Dispersion aqueuse de silicone selon la revendication 1 ou 2, caractérisée en ce que l'émulsion (A) a une teneur en extrait sec d'au moins 45 % en poids.

4. Dispersion aqueuse de silicone selon l'une quelconque des revendications précédentes, caractérisée en ce qu'elle comporte de 5 à 200 parties en poids de charge (D) choisie parmi l'alumine hydratée, l'alumine, le carbonate de calcium, la vermiculite expansée, la vermiculite non expansée, le noir de carbone, l'oxyde de zinc, le dioxyde de titane, le mica, le talc, l'oxyde de fer, le sulfate de baryum et la chaux éteinte.

5. Dispersion aqueuse de silicone selon la revendication 4, caractérisée en ce que le carbonate de calcium a un diamètre particulaire moyen inférieur à 0,1 μm.

6. Dispersion aqueuse de silicone selon l'une quelconque des revendications précédentes, caractérisé en ce qu'elle comporte :
(A) - 100 parties de l'émulsion du type huile dans eau d'un α,ω-(dihydroxy)polydiorganosiloxane de viscosité à 25 °C comprise entre 50 000 et 1 500 000 mPa.s et est stabilisée par un agent tensio-actif choisi parmi un sel de métal alcalin d'un acide hydrocarboné aromatique sulfonique et les alkylphénols polyoxyéthylénés,
(B) - 0,5 à 10 parties de silane,
(C) - 0,05 à 2 parties d'un dicarboxylate de diorganoétain,
(D) - 10 à 200 parties d'une charge minérale non siliceuse
ladite émulsion ayant un pH compris entre 8 et 13 et une teneur en extraits secs d'au moins 60 %.

7. Dispersion aqueuse de silicone selon l'une quelconque des revendications précédentes, caractérisée en ce qu'elle comporte en outre pour 100 parties d'une émulsion (A), un additif silicié (E), choisi parmi le silicate de soude (0,3 à 30 parties), et une charge siliceuse renforçante ou semi-renforçante (1 à 150 parties), sous réserve que pour 100 parties de (A), la somme des parties de (D) + (E) soit inférieure à 300 parties.

8. Dispersion aqueuse selon l'une quelconque des revendications précédentes, caractérisée en ce qu'elle comporte en outre, pour 100 parties d'émulsion (A), 1 à 40 parties en poids d'une résine silicone hydroxylée (F) présentant, par molécule, au moins deux motifs différents choisis parmi ceux de formules : $R_3SiO_{0,5}$, $R_2SiO$, $RSiO_{1,5}$ et $SiO_2$, les radicaux R, identiques ou différents, étant choisis parmi les radicaux vinyle, phényle, trifluoro-3,3,3 propyle et les radicaux alkyle linéaires ou ramifiés ayant de 1 à 6 atomes de carbone inclus, ladite résine ayant une teneur pondérale en groupe hydroxyle comprise entre 0,1 et 10 %.

9. Dispersion aqueuse selon l'une quelconque des revendications précédentes, caractérisée en ce que le pH de 7 à 13 est obtenu par addition d'une quantité adaptée d'une solution aqueuse d'une base minérale forte (G) choisie parmi les hydroxydes alcalins et alcalino-terreux.

10. Utilisation d'une dispersion aqueuse telle que définie à l'une quelconque des revendications 1 à 9 pour l'enduction et l'enrobage de principes actifs pharmaceutiques ou phytosanitaires et pour la réalisation de revêtements d'objets en contact avec des aliments.

11. Utilisation selon la revendication 10, caractérisée en ce que lesdits objets en contact avec des aliments sont des bouchons de liège pour le conditionnement de vins et spiritueux.

## Patentansprüche

1. Wäßrige Silicondispersion, die durch Wasserabspaltung unter Umgebungsbedingungen zu einem Elastomer vernetzt, dadurch gekennzeichnet, daß sie in Gewicht umfaßt:

(A) - 100 Gewichtsteile einer Emulsion vom Typ Öl-in-Wasser eines $\alpha,\omega$-(Dihydroxy)-polydiorganosiloxans, stabilisiert durch mindestens ein oberflächenaktives Mittel, ausgewählt unter den anionischen und den nichtionischen oberflächenaktiven Mitteln und ihren Mischungen,

(B) - 0,1 bis 20 Gewichtsteile eines Silans der Formel (1)

$$\begin{array}{c} (R^1)_b \\ | \\ (R^2O)_{4-(a+b)}Si - (X)_a \end{array} \qquad (1)$$

in der

- X eine hydrolysierbare Gruppe ist, ausgewählt unter einem Aminorest der Formel $R^3NH$- und einem Amidorest der Formel $R^4$-CO-N($CH_3$)-,
- $R^1$ einen einwertigen Kohlenwasserstoffrest von $C_1$ bis $C_{13}$ darstellt,
- $R^2$ einen aliphatischen, organischen Rest von $C_1$ bis $C_8$ bedeutet, ausgewählt unter den Alkylresten, den Alkylether-Resten, den Alkylester-Resten, den Cyanoalkyl-Resten oder einem Aralkyl-Rest von $C_7$ bis $C_{13}$,
- $R^3$ ein Alkylrest von $C_1$ bis $C_6$, ein Phenylrest oder ein Cyclohexylrest ist,
- $R^4$ einen Alkylrest von $C_1$ bis $C_6$ oder einen Phenylrest darstellt,
- a eine ganze Zahl von 2, 3 oder 4 ist,
- b eine ganze Zahl von 0 oder 1 ist,
- a + b gleich 2, 3 oder 4 ist,

(C) - 0 bis 3 Gewichtsteile einer metallischen, katalytischen Härter-Verbindung,

(D) - 0 bis 250 Gewichtsteile eines mineralischen, nichtsilikatischen Füllstoffes, wobei die genannte Dispersion einen pH-Wert von größer als 7 und einen Gehalt an Trockenextrakt von mindestens 40 % aufweist.

2. Wäßrige Dispersion nach Anspruch 1, dadurch gekennzeichnet, daß sie 0,01 bis 3 Teile metallische Verbindung (C) umfaßt, die ein Organo-Zinnsalz (D) ist, das in Form einer wäßrigen Emulsion vorliegt.

3. Wäßrige Silicondispersion nach Anspruch 1 oder 2, dadurch gekennzeichnet, daß die Emulsion (A) einen

Gehalt an Trockenextrakt von mindestens 45 Gew.-% aufweist.

4. Wäßrige Silicondispersion nach irgendeinem der vorstehenden Ansprüche, dadurch gekennzeichnet, daß sie 5 bis 200 Gewichtsteile an Füllstoff (D) umfaßt, ausgewählt unter hydratisierter Tonerde, Tonerde, Calciumcarbonat, ausgedehntem Vermiculit, nichtausgedehntem Vermiculit, Aktivkohle, Zinkoxid, Titandioxid, Glimmer, Talk, Eisenoxid, Bariumsulfat und gelöschtem Kalk.

5. Wäßrige Silicondispersion nach Anspruch 4, dadurch gekennzeichnet, daß das Calciumcarbonat einen mittleren Teilchendurchmesser von unterhalb 0,1 µm besitzt.

6. Wäßrige Silicondispersion nach irgendeinem der vorstehenden Ansprüche, dadurch gekennzeichnet, daß sie umfaßt:
   (A) - 100 Teile Emulsion vom Typ Öl-in-Wasser eines α,ω-(Dihydroxy) -polydiorganosiloxans mit einer Viskosität bei 25 °C zwischen 50.000 und 1.500.000 mPa·s und stabilisiert durch ein oberflächenaktives Mittel, ausgewählt unter einem Alkalimetallsalz einer aromatischen Kohlenwasserstoff-Sulfonsäure und den polyoxyethylenierten Alkylphenolen,
   (B) - 0,5 bis 10 Teile Silan,
   (C) - 0,05 bis 2 Teile eines Diorganozinn-Dicarboxylates,
   (D) - 10 bis 200 Teile eines mineralischen, nichtsilikatischen Füllstoffes,
   wobei die genannte Dispersion einen pH-Wert zwischen 8 und 13 und einen Gehalt an Trockenextrakten von mindestens 60 % aufweist.

7. Wäßrige Silicondispersion nach irgendeinem der vorstehenden Ansprüche, dadurch gekennzeichnet, daß sie außerdem pro 100 Teile Emulsion (A) umfaßt: einen siliciumhaltigen Zusatzstoff (E), ausgewählt unter Natriumsilikat (0,3 bis 30 Teile) und einem verstärkenden oder halb-verstärkenden silikatischen Füllstoff (1 bis 150 Teile), unter dem Vorbehalt, daß pro 100 Teile (A) die Summe der Teile (D) und (E) unterhalb von 300 Teilen beträgt.

8. Wäßrige Dispersion nach irgendeinem der vorstehenden Ansprüche, dadurch gekennzeichnet, daß sie außerdem pro 100 Teile Emulsion (A) umfaßt: 1 bis 40 Gewichtsteile eines hydroxylierten Siliconharzes (F), das pro Molekül mindestens zwei verschiedene Einheiten aufweist, ausgewählt unter denen der Formeln: $R_3SiO_{0,5}$, $R_2SiO$, $RSiO_{1,5}$ und $SiO_2$, wobei die Reste R, gleich oder verschieden, ausgewählt werden unter den Resten Vinyl, Phenyl, 3,3,3-Trifluor-propyl und den linearen oder verzweigten Alkylresten mit 1 bis einschließlich 6 Kohlenstoffatomen, und das genannte Harz einen gewichtsmäßigen Gehalt an Hydroxylgruppen zwischen 0,1 und 10 % aufweist.

9. Wäßrige Dispersion nach irgendeinem der vorstehenden Ansprüche, dadurch gekennzeichnet, daß der pH-Wert von 7 bis 13 durch Zugabe einer angepaßten Menge der wäßrigen Lösung einer starken Mineralbase (G) erhalten wird, ausgewählt unter den Alkalihydroxiden und den Erdalkalihydroxiden.

10. Verwendung einer wäßrigen Dispersion, wie in irgendeinem der Ansprüche 1 bis 9 definiert, für die Beschichtung und Umhüllung von pharmazeutischen und Pflanzenschutz-Wirkstoffen und für die Realisierung von Überzügen auf Gegenständen, die Kontakt mit Nahrungsmitteln haben.

11. Verwendung nach Anspruch 10, dadurch gekennzeichnet, daß die Gegenstände, die Kontakt mit Nahrungsmitteln haben, Korkpfropfen für die Abfüllung von Weinen und alkoholischen Getränken sind.

## Claims

1. Aqueous silicone dispersion crosslinking to an elastomer by elimination of water under ambient conditions, characterized in that it contains, by weight:
   (A) - 100 parts by weight of an oil-in-water type emulsion of an α,ω-(dihydroxy)polydiorganosiloxane stabilized by at least one surface-active agent chosen from anionic and nonionic surface-active agents and their mixtures,
   (B) - 0.1 to 20 parts by weight of a silane of formula:

$$\begin{array}{c} (R^4)_b \\ | \\ (R^2O)_{4-(a+b)}Si-(X)_a \end{array} \qquad (1)$$

in which

- X is a hydrolysable group chosen from an amino radical of formula $R^3NH$- and an amido radical of formula $R^4$-CO-N($CH_3$)-,
- $R^1$ is a $C_1$-$C_{13}$ monovalent hydrocarbon radical,
- $R^2$ is a $C_1$-$C_8$ aliphatic organic radical chosen from alkyl radicals, alkyl ether radicals, aklyl ester radicals, cyanoalkyl radicals or a $C_7$-$C_{13}$ aralkyl radical,
- $R^3$ is a $C_1$-$C_6$ alkyl, phenyl or cyclohexyl radical,
- $R^4$ is a $C_1$-$C_6$ alkyl or phenyl radical,
- a is an integer equal to 2, 3 or 4,
- b is an integer equal to 0 or 1,
- a + b is equal to 2, 3 or 4,

(C) - 0 to 3 parts by weight of a metallic cure catalyst compound,

(D) - 0 to 250 parts by weight of an inorganic nonsiliceous filler, the said dispersion having a pH higher than 7 and a solid extracts content of at least 40 %.

**2.** Aqueous dispersion according to Claim 1, characterized in that it contains 0.01 to 3 parts of metallic compound (C) which is an organotin salt (D) present in the form of an aqueous emulsion.

**3.** Aqueous silicone dispersion according to Claim 1 or 2, characterized in that the emulsion (A) has a solids content of at least 45 % by weight.

**4.** Aqueous silicone dispersion according to any one of the preceding claims, characterized in that it contains from 5 to 200 parts by weight of filler (D) chosen from hydrated alumina, alumina, calcium carbonate, expanded vermiculite, nonexpanded vermiculite, carbon black, zinc oxide, titanium dioxide, mica, talc, iron oxide, barium sulphate and slaked lime.

**5.** Aqueous silicone dispersion according to Claim 4, characterized in that the calcium carbonate has a mean particle diameter of less than 0.1 μm.

**6.** Aqueous silicone dispersion according to any one of the preceding claims, characterized in that it contains:

(A) - 100 parts of the oil-in-water type emulsion of an $\alpha,\omega$-(dihydroxy)polydiorganosiloxane with a viscosity at 25°C of between 50,000 and 1,500,000 mPas and is stabilized by a surface-active agent chosen from an alkali metal salt of an aromatic hydrocarbon sulphonic acid and polyoxyethylenated alkylphenols,

(B) - 0.5 to 10 parts of silane,

(C) - 0.05 to 2 parts of a diorganotin dicarboxylate,

(D) - 10 to 200 parts of an inorganic non-siliceous filler,

the said emulsion having a pH between 8 and 13 and a solids content of at least 60 %.

**7.** Aqueous silicone dispersion according to any one of the preceding claims, characterized in that it additionally contains, per 100 parts of an emulsion (A), a silicon-containing additive (E) chosen from sodium silicate (0.3 to 30 parts) and a reinforcing or semi-reinforcing siliceous filler (1 to 150 parts), with the proviso that, per 100 parts of (A), the sum of the parts of (D) + (E) is less than 300 parts.

**8.** Aqueous dispersion according to any one of the preceding claims, characterized in that it additionally contains, per 100 parts of emulsion (A), 1 to 40 parts by weight of a hydroxylated silicone resin (F) having, per molecule, at least two different units chosen from those of formulae: $R_3SiO_{0.5}$, $R_2SiO$, $RSiO_{1.5}$ and $SiO_2$, the radicals R, which may be identical or different, being chosen from vinyl, phenyl and 3,3,3-trifluoropropyl radicals and linear or branched alkyl radicals having from 1 to 6 carbon atoms inclusive, the said resin having a hydroxyl group weight content of between 0.1 and 10 %.

**9.** Aqueous dispersion according to any one of the preceding claims, characterized in that the pH of 7 to 13 is obtained by addition of a suitable quantity of an aqueous solution of a strong inorganic base (G) chosen from alkali metal and alkaline-earth metal hydroxides.

**10.** Use of an aqueous dispersion as defined in any one of Claims 1 to 9 for the covering and coating of pharmaceutical or plant-protection active principles and for the preparation of coatings for objects in contact with food.

**11.** Use according to Claim 10, characterized in that the said objects in contact with food are cork stoppers for the bottling of wines and spirits.